# EUROPÄISCHE PATENTSCHRIFT

(11) **EP 3 235 492 B1**
(45) Veröffentlichungstag und Bekanntmachung des Hinweises auf die Patenterteilung: **12.06.2019**
(21) Anmeldenummer: 16166222.6
(22) Anmeldetag: 20.04.2016
(51) Int. Cl.: A61K 9/46, A61K 9/00, A61K 47/10, A61K 9/08, A61K 9/16, A61K 9/20, A61K 31/167, A61K 47/22, A61K 31/352

(54) **VERWENDUNG VON HOMOERIODICTYOL (HED) ZUM REDUZIEREN DER MAGENSÄURESEKRETIONS-STIMULIERENDEN WIRKUNG VON N-ACETYL-4-AMINOPHENOL (PARACETAMOL)**
USE OF HOMOERIODICTYOL (HED) FOR REDUCING THE GASTRIC ACID SECRETION STIMULATING EFFECT OF N-ACETYL-4-AMINOPHENOL (PARACETAMOL)
UTILISATION D'HOMOERIODICTYOL (HED) DESTINE A REDUIRE L'EFFET DE STIMULATION DE LA SECRETION DE L'ACIDE GASTRIQUE DE N-ACETYL-4-AMINOPHENOL (PARACETAMOL)

(43) Veröffentlichungstag der Anmeldung: 25.10.2017
(73) Patentinhaber: Symrise AG, 37603 Holzminden (DE)
(72) Erfinder: HANS, Joachim, 37603 Holzminden (DE); LEY, Jakob Peter, 37603 Holzminden (DE); PAETZ, Susanne, 37671 Höxter (DE); MIDDENDORF, Silke, 49196 Bad Laer (DE); LISZT, Kathrin, 1200 Wien (AT); SOMOZA, Veronika, 3400 Weidling (AT)
(74) Vertreter: Eisenführ Speiser

(56) Entgegenhaltungen:
- EP-A2- 1 258 200
- WO-A1-2014/111436
- WO-A1-2014/111546

## Beschreibung

Die vorliegende Erfindung betrifft Homoeriodictyol (HED) oder ein Salz davon oder eine Mischung aus HED und einem oder mehreren Salzen davon oder eine Mischung aus mehreren Salzen davon zur Anwendung in einem therapeutischen Verfahren zum Reduzieren der Magensäuresekretions-stimulierenden Wirkung von N-Acetyl-4-aminophenol (Paracetamol).

Weitere sowie bevorzugte Aspekte der vorliegenden Erfindung ergeben sich aus der nachfolgenden Beschreibung samt Beispielen sowie insbesondere den beigefügten Patentansprüchen.

Paracetamol (*N*-Acetyl-4-aminophenol; *N*-Acetyl-p-aminophenol; 4-Acetaminophenol; Acetaminophen (USAN); APAP; 4'-Hydroxyacetanilid; p-Hydroxyacetanilid; IUPAC: *N*-(4-Hydroxyphenyl)acetamid; Latein: Paracetamolum; med. Abk.: PCM) ist ein schmerzstillender und fiebersenkender Arzneistoff aus der Gruppe der Nichtopioid-Analgetika und wird vielfältig eingesetzt. Paracetamol hat jedoch einen charakteristischen, leicht bitteren Geschmack, der als unangenehm empfunden werden kann. Zudem kann Paracetamol Reflux-Syndrome auslösen, indem die Magensäuresekretion stimuliert wird.

In EP 1 258 200 A2 wird die Verwendung von Hydroxyflavanonen wie Homoeriodictyol zur Maskierung oder Verminderung des bitteren Geschmackseindrucks von Bitterstoffen wie Paracetamol beschrieben.

Die Stimulation der Magensäuresekretion stellt generell einen wichtigen Mechanismus zur Einleitung der Verdauung von Nahrung, insbesondere proteinreicher Nahrung dar. Es kann durchaus positiv für die Verdauung sein, die Sekretion kurzfristig maßvoll zusätzlich anzuregen. Wird Magensäure aber übermäßig sekretiert und somit der pH im Magen zu stark gesenkt, so führt das akut in der Regel z.B. zu Unwohlsein oder saurem Aufstoßen. Wenn dieser Zustand länger anhält oder chronisch die Magensäuresekretion stark angeregt wird, so können entzündliche Zustände der Magenschleimhaut sowie der Speiseröhre induziert werden, die ihrerseits wiederum als Spätfolge Geschwüre und im schlimmsten Fall auch bösartige Gewebeveränderungen bis zum Magenkrebs und Speiseröhrenkrebs auslösen können.

In WO 2014/111436 wird eine *in vitro* Methode zur Identifizierung von bitter schmeckenden oder den bitteren Geschmack modulierenden Verbindungen über deren Effekt auf die Protonensekretion von HGT-1 Zellen beschrieben.

Aufgabe der vorliegenden Erfindung war es daher, Stoffe, bevorzugt natürliche Stoffe, anzugeben, die dazu geeignet sind, die Magensäuresekretions-stimulierende Wirkung von Paracetamol zu reduzieren. Vorzugsweise sollen Stoffe angegeben werden, die die Säuresekretion besonders stark reduzieren oder sogar vollständig verhindern können.

Herkömmlicherweise verwendet man zwei große Gruppen von Wirkstoffen zur Reduktion der Magensäuresekretion bzw. um dieser entgegenzuwirken, nämlich zum Einen (neutralisierende) basische Stoffe, wie zum Beispiel Natriumhydrogencarbonat, Calciumcarbonat, basische Aluminium- oder Magnesiumhydroxide, die den pH-Wert erhöhen, und zum Anderen Stoffe, die die Magensäuresekretion durch direkte Blockierung der auf den sekretorischen Zellen (Parietalzellen) zu findenden Acetylcholin-Rezeptoren (M3-Typ) oder öfter H2-Histaminrezeptoren reduzieren, zum Beispiel Pirenzepin, Cimetidin, Ranitidin oder Famotidin.

Eine weitere Möglichkeit, die Magensäuresekretion zu reduzieren, besteht in der direkten Modulation der ATP-getriebenen Protonenpumpe der Parietalzellen (s. z.B. Opremazol).

Überraschenderweise wurde nunmehr im Rahmen der vorliegenden Erfindung gefunden, dass Homoeriodictyol (HED; 3'-Methoxy-4',5,7-trihydroxyflavanon) nicht nur dazu in der Lage ist, den bitteren Geschmack von Paracetamol zu reduzieren, sondern auch, die Magensäuresekretions-stimulierende Wirkung von Paracetamol zu reduzieren, ja sogar vollständig zu verhindern.

Die vorliegende Erfindung betrifft daher HED oder ein Salz davon oder eine Mischung aus HED und einem oder mehreren Salzen davon oder eine Mischung aus mehreren Salzen davon zur Anwendung in einem therapeutischen Verfahren zum Reduzieren der Magensäuresekretions-stimulierenden Wirkung von Paracetamol.

Anwendungen im Rahmen eines therapeutischen Verfahrens sind vorzugsweise solche, bei denen die durch Homoeriodictyol (HED) oder eines Salzes davon oder eine Mischung aus HED und einem oder mehreren Salzen davon oder eine Mischung aus mehreren Salzen davon bewirkte Reduktion der Magensäuresekretions-Stimulation von Paracetamol dazu dient, entzündliche Zustände der Magenschleimhaut und der Speiseröhre sowie dadurch verursachte Geschwüre oder bösartige Gewebeveränderungen bis hin zum Magenkrebs und Speiseröhrenkrebs, die durch die alleinige Gabe von Paracetamol ausgelöst werden können, zu verhindern oder deren Schweregrad zu vermindern. Diese Art der therapeutischen Verwendung liegt vor, wenn Individuen eine Mischung aus HED und/oder ein oder mehrerer Salze davon und Paracetamol oder eine Zubereitung, enthaltend HED und/oder ein oder mehrere Salze davon und Paracetamol oft, d.h. über einen Zeitraum von vielen Tagen pro Jahr in hoher Dosis, als schmerzstillenden oder fiebersenkenden Arzneistoff verwenden. Ein Beispiel eines solchen Falles sind Patienten mit chronischen Schmerzen, die häufig auf die Einnahme eines Analgetikums wie Paracetamol zum Schmerzmanagement angewiesen sind. Die regelmäßige Einnahme von Paracetamol allein kann zu den oben beschriebenen krankhaften Veränderungen der Magenschleimhaut und der Speiseröhre führen. Diese können jedoch durch die zusätzliche Einnahme von HED oder eines Salzes davon oder einer Mischung aus HED und einem oder mehreren Salzen davon oder einer Mischung aus mehreren Salzen davon zu Paracetamol verhindert bzw. deren Ausbildung und Symptome vermindert werden.

Es ist hinreichend bekannt, dass HED bittermaskierende Wirkung besitzt. Weiterhin ist in WO 2014111436 A1 beschrieben, dass zum Beispiel HED in der Lage ist, die Magensäuresekretions-stimulierende Wirkung diverser Stoffe zu reduzieren, insbesondere von Coffein oder Theobromin. Weiterhin wurde in WO 2014111436 A1 gezeigt, dass eine Verabreichung von HED allein, ohne die Zugabe weiterer Magensäuresekretions-stimulierender Stoffe, zu keiner signifikanten Wirkung auf die Protonensekretion in HGT-1-Zellen führt. Es wird vorliegend angenommen, dass diese Wirkung in Gegenwart Magensäuresekretions-stimulierender Stoffe über die Modulation von Bitterrezeptoren erfolgt. Aus der Literatur (W. Meyerhof et al., Chem. Senses 2010, 35, 157-170) ist bekannt, dass Coffein mehrere Bitterrezeptoren, z.B. TAS2R43, aktivieren kann. Aus eigenen Untersuchungen ist bekannt, dass HED als Antagonist auf eben diesen Rezeptortyp wirkt.

Dennoch ist überraschend, dass gerade HED besonders gut dazu geeignet ist, die Magensäuresekretions-stimulierende Wirkung von Paracetamol zu reduzieren. Denn HED wird auch als Agonist am Rezeptortyp TAS2R39 beschrieben (W. S. U. Roland et al., J. Agric. Food Chem. 2013, 61, 10454-10466). TAS2R39 ist der einzige für Paracetamol bekannte aktivierbare Bitterrezeptor. Nach den bisherigen Erkenntnissen war es also insofern nicht voraussehbar, sondern vielmehr besonders überraschend, dass die Kombination eines reinen TAS2R39-Agonisten (Paracetamol) und einer - unter anderem - ebenfalls TAS2R39 aktivierenden (aber andere Bitterrezeptoren inhibierenden) Verbindung (HED) im Ergebnis eine solche Wirkung wie hierin beschrieben zeigt. Wie sich im Rahmen eigener Untersuchungen herausgestellt hat, ist HED nicht nur in der Lage, die Magensäuresekretions-stimulierende Wirkung von Paracetamol zu reduzieren, sondern diese sogar vollständig zu unterdrücken. Darüber hinaus ist HED überraschenderweise sogar dazu in der Lage, eine signifikante Absenkung der konstitutiven Säuresekretion zu bewirken (vgl. hierzu die im Beispielteil wiedergegeben Versuchsergebnisse).

Ein möglicher Erklärungsansatz für die Eigenschaft von HED, die Magensäuresekretions-stimulierende Wirkung von Paracetamol in solchem Maße zu reduzieren, könnte sein, dass HED und Paracetamol kompetitiv an den gleichen Rezeptor, den Bitterrezeptor TAS2R39, binden. Roland et al. (PLoS ONE 2015, 10(3), e0118200) haben in ihrer strukturbasierten Pharmakophormodellierung mithilfe der Snooker Software wichtige Strukturmerkmale von (Iso)Flavonoid-basierten Agonisten und Antagonisten des TAS2R39-Rezeptors ermittelt. Dies ist besonders unter dem Aspekt interessant, dass bis heute keine Kristallstruktur irgendeines Bitterrezeptors erhalten werden konnte. Roland *et al.* gingen dabei davon aus, dass TAS2R39-Antagonisten an den gleichen Rezeptor wie TAS2R39-Agonisten binden und dabei die Rezeptoraktivierung der Agonisten vermindern oder aufheben, da sie zu keiner Rezeptoraktivierung fähig sind. Das Ergebnis der besagten Studie war, dass Antagonisten strukturell dadurch gekennzeichnet sind, dass sie keine Kohlenstoff-Kohlenstoff-Doppelbindung zwischen den Atomen 2 und 3 der Subklasse der Flavanone besitzen. Dies führt zu einer tetrahedralen (gewinkelten) Konformation an Position 2 für Antagonisten, wohingegen die meisten Agonisten eine Kohlenstoff-Kohlenstoff-Doppelbindung besitzen und daher eine planare Struktur aufweisen und tiefer in die Bindungstasche des TAS2R39-Rezeptors eindringen können. Zudem waren die getesteten Flavanone nur als Antagonisten wirksam, wenn sie eine Methoxygruppe an Atom 6 vorwiesen. Die Antagonisten hatten des Weiteren keine Wasserstoffbrücken-Donoren in ihrer Struktur, wohingegen die getesteten Agonisten zumindest eine Position mit einem Wasserstoffbrücken-Donor enthielten.

Die vorliegenden Ergebnisse von Roland *et al.* deuten darauf hin, dass es sich bei HED strukturell gesehen um ein "Hybridmolekül" in Bezug auf seine agonistischen und antagonistischen Wirkungen am TAS2R39-Rezeptor handelt. HED besitzt keine Kohlenstoff-Kohlenstoff-Doppelbindung zwischen den Atomen 2 und 3, d.h. es hat eine gewinkelte Struktur und kann vermutlich genauso wie die beschriebenen Antagonisten nicht besonders tief in die Bindungstasche des Rezeptors vordringen. Es besitzt allerdings keine Methoxy-Gruppe an Position 6 und hat drei Hydroxylgruppen zur Ausbildung von Wasserstoffbrückenbindungen, was dahingegen eher für eine agonistische Aktivität am TAS2R39-Rezeptor spricht.

Das Vorliegen von drei Hydroxylgruppen in der Struktur von HED könnte eventuell dazu führen, dass HED kompetitiv zu Paracetamol an den TAS2R39-Rezeptor bindet und dabei durch die stärkere Fähigkeit Wasserstoffbrücken auszubilden eine größere Bindungsaffinität als Paracetamol zum TAS2R39-Rezeptor aufweist. Gleichzeitig kann HED durch seine gewinkelte Struktur allerdings vermutlich nicht wie die planaren Agonisten tief in die Bindungstasche des Rezeptors vordringen und so keine besonders stark ausgeprägte agonistische Wirkung entfalten.

Im Hinblick auf die Beschreibung von HED in der Literatur als TAS2R39-Agonist und seine Struktur, die hauptsächlich agonistisch wirkende Elemente enthält, ist die starke Reduktion der Magensäuresekretions-stimulierenden Wirkung von Paracetamol durch HED ein unerwarteter Effekt, insbesondere in dem hierin beschriebenen Maße.

Im Rahmen eigener Untersuchungen hat sich gezeigt, dass die durch die Verwendung einer Konzentration von 3 mM Paracetamol induzierte Protonensekretion in humanen Parietalzellen (HGT-1-Zellen, human gastric tumor cell line) durch die Zugabe von 0,003 bzw. 0,03 mM HED vermindert wird und durch die Zugabe von 0,3 mM HED eine signifikante Absenkung der konstitutiven Säuresekretion bewirkt werden kann. D.h. bei einer Zugabe von 0,3 mM HED kann die durch die Gabe von 3 mM Paracetamol induzierte Protonensekretion in HGT-1-Zellen nicht nur vermindert bzw. aufgehoben werden, es wird in diesem Fall überraschenderweise vielmehr weniger Säure von den HGT-1-Zellen sekretiert als im Kontrollexperiment, welches ohne die Zugabe aktiver Substanzen zu den Zellen durchgeführt wird (Überkompensation).

Weiterhin ist es bevorzugt, wenn die Anwendung zudem ein Maskieren des bitteren Geschmacks von Paracetamol umfasst.

Ein bitterer Geschmack eines oral zu verabreichenden Wirkstoffes kann die Akzeptanz des Medikaments, das diesen Wirkstoff enthält, und die Compliance von Individuen stark negativ beeinträchtigen. Daher ist es wichtig, Stoffe bereitzustellen, die den bitteren Geschmack einer Substanz in einer den Bitterstoff enthaltenden Mischung oder Zubereitung maskieren.

Unter Maskieren wird im Rahmen des vorliegenden Textes eine Reduzierung, d.h. eine Verminderung, oder eine vollständige Unterdrückung verstanden. Das Maskieren des bitteren Geschmackseindrucks bedeutet damit im Ergebnis regelmäßig eine Geschmacksverbesserung, insbesondere in Bezug auf bittere Geschmackseindrücke.

Die besagte Geschmacksverbesserung kann durch verschiedene Strategien erreicht werden. Traditionell wurden unangenehme, bittere Geschmackseindrücke durch die Zugabe von angenehm empfundenen Geschmacksstoffen maskiert. Dabei handelt es sich um ein bloßes Überdecken des bitteren Geschmackseindruckes. Ein zweiter Ansatz, einen bitteren Geschmackseindruck eines Bitterstoffes zu vermindern oder vollständig zu unterdrücken, ist den Kontakt des Bitterstoffes mit den Bitterrezeptoren zu verhindern, zum Beispiel durch Einkapselung, molekularen Einschluss oder Komplexierung. Eine dritte Strategie des Maskierens ist die Verwendung sogenannter Bitter-Blockierer (Antagonisten). Für den TAS2R39-Rezeptor wurde zum Beispiel gefunden, dass 6-Methoxyflavanon die Reaktion des Rezeptors auf diverse Bitterstoffe (Agonisten) verringern kann (Roland et al., 6-Methoxyflavanones as Bitter Taste Receptor Blockers for hTAS2R39, PLoS ONE 2014, 9(4), e94451). 6-Methoxyflavanon weist dabei gemäß Roland *et al.* (2015) eindeutig eine Struktur auf, die mit TAS2R39-Rezeptor Antagonisten in Verbindung gebracht wird, d.h. es besitzt keine Kohlenstoff-Kohlenstoff-Doppelbindung zwischen den Atomen 2 und 3 (gewinkelte Struktur), es besitzt eine Methoxy-Gruppe an Position 6 und keine Wasserstoffbrücken-Donorgruppen.

Gemäß einem Aspekt der vorliegenden Erfindung sind HED und Paracetamol im Rahmen einer erfindungsgemäßen Anwendung gemeinsam in einer Zubereitung enthalten. D.h., bevorzugt ist demnach eine Anwendung (wie oben beschrieben) in einer Zubereitung, enthaltend HED und/oder ein oder mehrere Salze davon und Paracetamol, wobei die Menge an HED und/oder Salz(en) davon ausreicht, um die Magensäuresekretions-stimulierende Wirkung von Paracetamol zu reduzieren und, vorzugsweise, zudem ausreicht, um den bitteren Geschmack von Paracetamol zu maskieren.

Geeignete Zubereitungen sind beispielsweise solche, die in einer Form ausgebildet sind, die ausgewählt ist aus der Gruppe bestehend aus: Tabletten (nichtüberzogene sowie überzogene Tabletten, ein- oder mehrschichtige Tabletten), Kapseln, Dragees, Granulaten, Pellets, Feststoffmischungen, Dispersionen in flüssigen Phasen, Emulsionen, Pulver, Lösungen, Säfte, Pasten oder andere schluck- oder kaubare Zubereitungen, vorzugsweise solche die ausgewählt sind aus der Gruppe bestehend aus Tabletten, Kapseln, Säften, Kaugummis oder Fruchtgummis.

Weitere übliche Wirk-, Grund-, Hilfs- und Zusatzstoffe können in der Zubereitung in Mengen von 0,9 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, enthalten.

Die Zubereitung umfasst vorzugsweise zudem einen oder mehrere Bestandteile ausgewählt aus der Gruppe bestehend aus Eriodictyol, Phloretin, Hesperetin, 2,4-Dihydrobenzoesäure-*N*-Vanillylamid, 5,7-Dihydroxy-4-(4-hydroxy-phenyl)-chroman-2-on, 5,7-Dihydroxy-4-(4-hydroxy-3-methoxy-phyenyl)-chroman-2-on, 5,7- Dihydroxy-4-(4-pyridyl)-chroman-2-on, 7,3-Dihydroxy-4'-methoxyflavan, Lariciresinol und Matairesinol, und deren jeweiligen (wenn anwendbar) einzelnen oder in Gemischen vorliegenden Stereoisomeren (Diasteromere oder Enantiomere).

Das HED oder ein Salz davon oder eine Mischung aus HED und einem oder mehreren Salzen davon oder eine Mischung aus mehreren Salzen davon und Paracetamol können im Rahmen der vorliegenden Erfindung einem Individuum entweder gleichzeitig in einer gemeinsamen Zubereitung verabreicht werden oder sie können zeitlich versetzt voneinander in verschiedenen Zubereitungen verabreicht werden. D.h. das HED und/oder ein oder mehrere Salz(e) davon kann/können einem Individuum zeitlich vor, während oder nach der Gabe von Paracetamol verabreicht werden. Vorzugsweise werden das HED und/oder ein oder mehrere Salz(e) davon und das Paracetamol einem Individuum gleichzeitig in der gleichen Zubereitung verabreicht.

Bei gleichzeitiger Verabreichung von HED und/oder eines oder mehrerer Salz(e) davon und Paracetamol an ein Individuum, d.h. Formulierung selbiger Bestandteile in derselben Zubereitung, können das HED und/oder ein oder mehrere Salz(e) davon und das Paracetamol in vermischter Form vorliegen (z.B. in einer einschichtigen Tablette) oder sie können in getrennten Schichten vorliegen (z.B. in einer zwei- oder mehrschichtigen Tablette). Die Freisetzung des HED und/oder ein oder mehrerer Salz(e) davon und des Paracetamols kann dabei in zwei- oder mehrschichtigen Tabletten (oder anderen Formulierungsformen) nach oraler Gabe wiederum gleichzeitig oder zeitlich versetzt erfolgen. Vorzugsweise erfolgt die Freisetzung von HED und/oder eines oder mehrerer Salz(e) davon vor der Freisetzung des Paracetamols oder gleichzeitig mit der Freisetzung des Paracetamols.

Im Lichte der hierin beschriebenen Ergebnisse zu eigenen Untersuchungen zur Wirkung von HED (s. Beispielteil) ist es im Rahmen der vorliegenden Erfindung besonders bevorzugt, wenn in der Zubereitung die Magensäuresekretions-stimulierende Wirkung von Paracetamol durch HED und/oder das/die Salz(e) davon im Vergleich zu einer identischen Zubereitung ohne HED und/oder Salz(en) davon um wenigstens 10 % reduziert wird, vorzugsweise um wenigstens 20 %, weiter bevorzugt um wenigstens 50 %, besonders bevorzugt um wenigstens 70 %, ganz besonders bevorzugt vollständig.

In einer ganz besonders bevorzugten Ausführungsform wird die Magensäuresekretions-stimulierende Wirkung von Paracetamol durch HED und/oder das/der Salz(e) davon nicht nur stark reduziert, sondern es kommt zu einer signifikanten Absenkung der konstitutiven Säuresekretion der HGT-1-Zellen (Überkompensation), d.h. dass im Rahmen des im Beispielteil beschriebenen Zellassays (vgl. **Tabelle 1**) der Säuresekretionsspiegel der HGT-1-Zellen nach der Gabe von Paracetamol und HED und/oder eines oder mehrerer Salz(e) davon niedriger ist als bei einer unbehandelten Kontrolle, welche weder Paracetamol noch HED ausgesetzt war. In diesem Fall wird in der Zubereitung die Magensäuresekretions-stimulierende Wirkung von Paracetamol durch HED und/oder das/der Salz(e) davon im Vergleich zu einer identischen Zubereitung ohne HED und/oder Salz(en) davon um wenigstens 150 % reduziert, besonders bevorzugt um wenigstens 200 %, ganz besonders bevorzugt um bis zu 300 %.

Zu Details in Bezug auf die experimentelle Bestimmung der Reduktion der Magensäuresekretions-stimulierenden Wirkung von Paracetamol durch HED und/oder Salz(en) davon im Vergleich zu einer identischen Zubereitung ohne HED und/oder Salz(en) davon wird auf den unten folgenden Beispielteil verwiesen.

Bezüglich bevorzugter Mengen von HED gilt, dass die Konzentration von HED und/oder Salz(en) davon in der Zubereitung (wie oben beschrieben) im Bereich von 0,000001 bis 30 mM liegt, vorzugsweise im Bereich von 0,00001 bis 3 mM, weiter bevorzugt im Bereich von 0,0001 bis 1 mM, besonders bevorzugt im Bereich von 0,001 bis 0,4 mM.

Bezüglich bevorzugter Mengen von Paracetamol gilt, dass die Konzentration von Paracetamol in der Zubereitung im Bereich von 0,001 bis 1000 mM liegt, vorzugsweise im Bereich von 0,01 bis 800 mM, weiter bevorzugt im Bereich von 0,1 bis 750 mM, besonders bevorzugt im Bereich von 1 bis 600 mM.

Weiterhin ist es bevorzugt, wenn das Verhältnis der Gesamtkonzentration an HED und/oder Salz(en) davon zur Gesamtkonzentration an Paracetamol im Rahmen einer erfindungsgemäßen Anwendung, insbesondere in einer Zubereitung (wie oben beschrieben) im Bereich von 1 : 100000 bis 1 : 1 liegt, vorzugsweise im Bereich von 1 : 10000 bis 1 : 2, weiter bevorzugt im Bereich von 1 : 5000 bis 1 : 10, besonders bevorzugt im Bereich von 1 : 2000 bis 1 : 20.

HED bzw. Salze davon kommen in einer Vielzahl von Pflanzen vor. Die Hauptquelle sind die zu der Familie der Hydrophyllaceae zählenden Eriodictyon ssp., besser bekannt unter dem Namen Herba Santa oder Yerba Santa. Verfahren zur Bereitstellung bzw. zum Isolieren von HED und Salzen davon, aber auch andere Möglichkeiten, HED und dessen Salze zu erhalten, sind dem Fachmann inzwischen hinlänglich bekannt.

Sofern im Rahmen einer erfindungsgemäßen Anwendung ein oder mehrere Salze von HED verwendet werden, gilt, dass das bzw. ein, mehrere oder sämtliche Salze von HED vorzugsweise ausgewählt ist bzw. sind aus der Gruppe bestehend aus Natrium-, Kalium-, Calcium- und Ammonium-Salzen.

Im Kontext der vorliegenden Anmeldung werden auch folgende Verfahren beschrieben: Ein Verfahren zum Reduzieren der Magensäuresekretions-stimulierenden Wirkung von Paracetamol in einer oral konsumierbaren pharmazeutischen Zubereitung, umfassend den Schritt des Hinzufügens von HED oder eines Salzes davon oder einer Mischung aus HED und einem oder mehreren Salzen davon oder einer Mischung aus mehreren Salzen davon zu einer pharmazeutischen Zubereitung enthaltend Paracetamol in einer Menge, die ausreicht, um die Magensäuresekretions-stimulierende Wirkung von Paracetamol zu reduzieren und, vorzugsweise, zudem ausreicht, um den bitteren Geschmack von Paracetamol zu maskieren.

Bezüglich bevorzugter Mengenangaben bzw. Konzentrationen von HED und/oder eines oder mehrerer Salze(s) davon und von Paracetamol, Mengen- bzw. Konzentrationsverhältnisse selbiger zueinander und zu weiteren gegebenenfalls vorhandenen Bestandteilen in der Zubereitung gilt das oben Gesagte entsprechend.

Des Weiteren wird ein Verfahren beschrieben zum Reduzieren der MagensäureSekretion in einem Individuum, das Paracetamol verabreicht bekommt, umfassend den Schritt dem Individuum vor, nach oder gleichzeitig zu Paracetamol HED oder ein Salz davon oder eine Mischung aus HED und einem oder mehrerer Salz(e) davon oder eine Mischung aus mehreren Salzen davon zu verabreichen, in einer Menge, die ausreicht, um die Magensäuresekretions-stimulierende Wirkung von Paracetamol zu reduzieren und, vorzugsweise, zudem ausreicht, um den bitteren Geschmack von Paracetamol zu maskieren.

Nachfolgend wird die vorliegende Erfindung anhand ausgewählter Beispiele näher erläutert.

### Beispiele:

### Untersuchungen zur Wirkung von HED:

### Zellmodell

Als Zellmodell wurden humane Parietalzellen (human gastric tumor cell line, HGT-1) verwendet. Diese wurden von Dr. C. Laboisse (Laboratory of Pathological Anatomy, Nantes, France) zur Verfügung gestellt. Die Zellen werden unter den Standardbedingungen bei 37 °C, 5 % CO₂ in DMEM (Dulbecco's Modified Eagel's Medium) mit 4 g/L Glucose, 10 % FBS, 2 % L-Glutamin und 1 % Penicillin/Streptomycin kultiviert. Für die Messung der intrazellulären Protonenkonzentration werden die Zellen mit Trypsin/EDTA behandelt, die Zellviabilität mittels Tryptanblaufärbung bestimmt und 100 000 Zellen/well in schwarzen 96-well Platten ausgesät.

### Bestimmung des intrazellulären pH-Wertes in HGT-1-Zellkulturen

Zur Messung des intrazellularen pH-Wertes in HGT-1-Zellkulturen wird der Farbstoff 1,5-Carboxy-Seminaphtarhodafluor-Acetoxymethylester (SNARF-1-AM) verwendet. Die Zellen in den 96-well Platten werden einmal mit Krebs-Hepes-Puffer (KRHP) gewaschen und mit dem Farbstoff in der Konzentration von 3 µM gelöst in KRHP für 30 min bei 37 °C und 5 % CO₂ inkubiert. Danach werden die Zellen zwei Mal mit KRHP gewaschen und mit Magensäuresekretions-stimulierenden Stoffen, beispielsweise 3 mM Coffein, 0,3 mM Theobromin oder 3 mM Paracetamol allein oder in Kombination mit Stoffen zur Reduktion der Magensäure-stimulierenden Wirkung der vorangehenden Stoffe, beispielsweise Homoeriodictyol (HED), in unterschiedlichen Konzentrationen in phenolrotfreiem DMEM im Volumen von 100 µL aufgetragen; als weiteres Kontrollexperiment werden die oben genannten Magensäuresekretions-stimulierenden Stoffe alleine geprüft. Homoeriodictyol wird in doppelt destilliertem Wasser gelöst. Die finale Konzentration an Lösungsmittel, die den Zellen zugeführt wird, beträgt maximal 1 %. Der Fluoreszenzfarbstoff wird bei der Wellenlänge von 488 nm angeregt und die Emission bei 580 nm und 640 nm gemessen. Das Verhältnis der Fluoreszenzwerte von 580 nm zu 640 nm wird aufgetragen im Vergleich zur Kalibrierkurve, woraus der pH-Wert ermittelt werden kann. Für die Kalibrierkurve werden die Zellen mit einem Kaliumpuffer unterschiedlicher pH-Werte von 7,2-8,2 und 2 µM Nigericin behandelt. Nigericin equilibriert den intrazellulären und extrazellulären pH-Wert, sodass der intrazelluläre pH-Wert definiert werden kann. Die intrazelluläre H⁺ - Konzentration wird aus dem intrazellulären pH-Wert ermittelt. Der intrazelluläre Protonenindex (IPX) wird kalkuliert durch log2-Transformation des Verhältnisses zwischen behandelten Zellen und unbehandelten Zellen (Kontrolle) (Rubach, M.; Lang, R.; Seebach, E.; Somoza, M.M.; Hofmann, T.; Somoza, V., Mol Nutr Food Res 2012, 56, 325-335; Rubach, M.; Lang, R.; Hofmann, T.; Somoza, V., Ann N Y Acad Sci 2008, 1126, 310-4; Rubach, M.; Lang, R.; Skupin, C.; Hofmann, T.; Somoza, V., J Agric Food Chem 2010, 58, 4153-61; Weiss, C.; Rubach, M.; Lang, R.; Seebach, E.; Blumberg, S.; Frank, O.; Hofmann, T.; Somoza, V., J Agric Food Chem 2010, 58, 1976-85; Liszt, K. I.; Walker, J.; Somoza, V., J Agric Food Chem 2012, 60, 7022-7030; Walker, J.; Hell, J.; Liszt, K. I.; Dresel, M.; Pignitter, M.; Hofmann, T.; Somoza, V., J Agric Food Chem 2012, 60, 1405-12).

Die Anzahl der angegebenen Replikate beziehen sich auf technische Replikate (tr) oder auf die Anzahl an Gesamtreplikaten (n), die sich aus der Anzahl der technischen Replikate multipliziert mit der Anzahl an biologischen Replikaten ergibt.

In der folgenden **Tabelle 1** ist die gemessene prozentuale Steigerung (positive Werte) bzw. prozentuale Reduktion (negative Werte) der Protonensekretion in HGT-1-Zellen nach 10 minütiger Stimulierung durch 3 mM Coffein, 0,3 mM Theobromin oder 3 mM Paracetamol allein oder in Kombination mit Homoeriodictyol (HED) in unterschiedlichen Konzentrationen angegeben. Die Daten sind dargestellt als Mittelwert und mittlere Standardabweichung, n=3-5, tr=6. Statistik: one-way Anova mit Holm-Sidak post-hoc Test. Signifikante Unterschiede (p < 0,05) werden durch Buchstaben angegeben.

**Tabelle 1**

| Beeinflussung der Paracetamol-induzierten Protonensekretion von HGT-1 Zellen durch HED im Vergleich zur Beeinflussung der Theobromin- und Coffein-induzierten Protonensekretion (wie in WO 2014111436 A1 beschrieben): | | | | | | |
|---|---|---|---|---|---|---|
| | **Coffein 3 mM** | | **Theobromin 0.3 mM** | | **Paracetamol 3 mM** | |
| | **MW** | **SEM** | **MW** | **SEM** | **MW** | **SEM** |
| DMEM | 54,80 | 5,61 | 20,92 | 3,37 | 24,10 | 2,23 |
| + 0,003 mM HED | | | 21,56 | 2,33 | 10,96 | 5,12 |
| + 0,03 mM HED | 38,00 | 6,70 | 13,79 | 3,50 | 6,57 | 4,23 |
| + 0,3 mM HED | 20,20 | 5,99 | -16,92 | 3,27 | -46,24 | 3,41 |

**Tabelle 1** ist zu entnehmen, dass es im Falle einer Anregung der Protonensekretion in HGT-1-Zellen durch 3 mM Coffein allein zu einer Zunahme der Protonensekretion von 55 % kommt. Die Beigabe von HED mildert die Coffein-induzierte Zunahme der Protonensekretion etwas ab, d.h. bei gleichzeitiger Inkubation der Zellen mit 3 mM Coffein und 0,03 mM HED steigert sich die Protonensekretion nur um 38 % und bei gleichzeitiger Inkubation der Zellen mit 3 mM Coffein und 0,3 mM HED steigert sich die Protonensekretion nur um 20 %.

Im Falle einer Anregung der Protonensekretion in HGT-1-Zellen durch 0,3 mM Theobromin allein kommt es zu einer Zunahme der Protonensekretion von 21 %. Die Beigabe von HED mildert die Theobromin-induzierte Zunahme der Protonensekretion etwas ab, d.h. bei gleichzeitiger Inkubation der Zellen mit 0,3 mM Theobromin und 0,03 mM HED steigert sich die Protonensekretion nur um 14 %. Bei gleichzeitiger Inkubation der Zellen mit 0,3 mM Theobromin und 0,3 mM HED reduziert sich die Protonensekretion sogar um 17 %.

Im Falle einer Anregung der Protonensekretion in HGT-1-Zellen durch 3 mM Paracetamol allein kommt es zu einer Zunahme der Protonensekretion von 24 %. Die Beigabe von HED mildert die Zunahme der Protonensekretion nach Paracetamol-Gabe deutlich ab. Bei gleichzeitiger Inkubation der Zellen mit 0,3 mM Paracetamol und nur 0,003 mM HED steigert sich die Protonensekretion um nur 11 %. Bei gleichzeitiger Inkubation der Zellen mit 3 mM Paracetamol und 0,03 mM HED steigert sich die Protonensekretion um nur 7 %. Bei gleichzeitiger Inkubation der Zellen mit 3 mM Paracetamol und 0,3 mM HED reduziert sich die Protonensekretion deutlich um 46 %.

### Anwendungsbeispiele:

### Anwendungsbeispiel 1: HED-Darreichungsformen

HED-1: Homoeriodictyol, Reinheit 95% (Firma Interquim, Spanien)
HED-2: Homoeriodictyol Natriumsalz Variante 1:
   10 g Homoeriodictyol (95%) werden im Kolben vorgelegt und 44 g NaOH-Lösung 3%ig dazugeben. Die zuerst dunkelbraune Lösung wird schnell breiartig, dann mit Wasser verdünnt bis zur homogenen Suspension und 1 Stunde nachgerührt und dann gefriergetrocknet oder sprühgetrocknet. Ausbeute: 10,44 g; HPLC: 94,2% HED-Na gegen Standard
HED-3: Homoeriodictyol Natriumsalz Variante 2:
   10 g Homoeriodictyol (95%) werden in 100 ml Ethylacetat suspendiert und 44 g NaOH-Lösung 3%ig dazugegeben. Die zunächst klare Lösung wird trüb und das Produkt fällt aus. Nach 1 Stunde Rühren bei Raumtemperatur wird die Suspension über eine Drucknutsche filtriert und der Filterrückstand im Vakuumtrockenschrank getrocknet. Ausbeute: 9,63g; HPLC: 98,5% HED-Na gegen Standard.
HED-4: Homoeriodictyol Natriumsalz, natürlich, wurde nach WO 2004/041804 hergestellt.
HED-5: 60.8 Gew.-% Wasser werden vorgelegt und 6.1 Gew.-% Gummi Arabicum sowie 24.3 Gew.-% Maltodextrin (aus Maisstärke) aufgelöst. 8.8 Gew.-% des Homoeriodictyolnatriumsalz (HED-2, HED-3 oder HED-4) werden hinzugefügt und mit einem Ultraturrax oder einem anderen Homogenisator aufgemischt. Die emulgierte Suspension wird dann in einem Sprühturm (Inlet-Temperatur 185-195°C, Outlet-Temperatur 70-75 °C) sprühgetrocknet und man erhält ein mit ca. 18-22 % Homoeriodictyol beladenes Sprühprodukt.
HED-6: 2.5 Gew.-% Homoeriodictyol (HED-1) werden in 1,2-Propylenglycol gelöst
HED-7: 1 Gew.-% Homoeriodictyolnatriumsalz (HED-2, HED-3 oder HED-4) werden in Ethanol gelöst
   In den folgenden Anwendungen werden nur Inhaltsstoffe von Pharmaqualität verwendet.

### Anwendungsbeispiel 2: Sachets

3,00 g wasserfreie Zitronensäure
2,50 g Aspartam
1,00 g Ascorbinsäure
82,00 g Saccharose
10,00 g Paracetamol-Pulver
1,50 g HED-5 (sprühgetrocknetes HED-Natriumsalz auf Maltodextrin)

Die Zutaten werden gemischt und anschließend in 5 g-Portionen abgepackt.

Dosierung: 5 g Pulver werden in 100 ml gegeben und appliziert.

### Anwendungsbeispiel 3: Brausetabletten

50,00 g Paracetamol
27,00 g Sorbitol
5,00 g Sodiumcyclamat
0.80 g Saccharin
5.00 g HED-5 (sprühgetrocknetes HED-Natriumsalz auf Maltodextrin)
2,00 g 1,2-Propylenglycol

Die Komponenten werden gemischt und anschließend
100,00 g Natriumhydrogencarbonat
136,00 g Zitronensäure
zugegeben. Nach 2 Stunden werden daraus Tabletten gepresst (2 g / Tablette).
Dosierung: 1 Tablette wird in 100 ml Wasser gegeben und appliziert.

### Anwendungsbeispiel 4: Saft

5 ml Saft enthalten:
200 mg Paracetamol
20 mg HED-1
Sonstige Bestandteile: Gereinigtes Wasser, Sucrose, Natriumcitrat, Tragant, Citronensäure, Methyl-4-hydroxybenzoat (E218), Propyl-4-hydroxybenzoat (E216).

### Anwendungsbeispiel 5: Pulver zum Herstellen einer Lösung:

1 Beutel (6g) enthält:
600 mg Paracetamol
80 mg HED-2 (Homoeriodictyol Natriumsalz Variante 1)
Sonstige Bestandteile: Citronensäure, Natriumcitrat, Saccharose 3.7g, Saccharin-Natriumsalz, Natriumcyclamat, Maisstärke, hochdisp. Siliciumdioxid, Ascorbinsäure, Maltodextrin, Butylhydroxyanisol, modifizierte Stärke, Curcumin, Orangenaroma.

### Anwendungsbeispiel 6: Brausetablette (Effervescent)

1 Brausetablette enthält:
500 mg Paracetamol
100 mg HED-5
Sonstige Bestandteile: Ascorbinsäure, Citronensäure, Lactose 1H2O, Macrogol 6000, Methylcellulose, Natriumcyclamat, Natriumhydrogencarbonat, Natriumsulfat, Povidon K25, Saccharin-Natriumsalz, Simeticon, Sorbinsäure, Zitronenaroma

### Anwendungsbeispiel 7: Saft

1 Flasche (100ml) enthält:
4.000 mg Paracetamol, d.h. eine Dosis (5 ml) enthalten 200 mg Paracetamol.
200 mg HED-3 (Homoeriodictyol Natriumsalz Variante 2)
Sonstige Bestandteile: Natriummetabisulfit (E223), Propylenglycol, Saccharin-Natriumsalz, Sorbitol Lsg. 70% (nicht kristall.) (E420), ger. Wasser, Aroma Typ Kirsche

### Anwendungsbeispiel 8: Tabletten

1 Tablette enthält:
50 mg Paracetamol
10 mg HED-5
Sonstige Bestandteile: Maisstärke, Povidon, Talkum, Croscarmellose-Natrium, Mikrokristalline Cellulose, Magnesiumstearat

### Anwendungsbeispiel 9: Lösung

100 ml Lösung enthalten:
4000 mg Paracetamol
8000 mg HED-6 (2.5 %ige Lösung von HED-1 in 1,2-Propylenglycol)
Sonstige Bestandteile: Glycerol, Natriummetabisulfit, Citronensäure, Natriumhydroxid, Acesulfam-Kaliumsalz, Aroma Typ Rotfrucht, Macrogol, Saccharin-Natriumsalz, gereinigtes Wasser

### Anwendungsbeispiel 10: Tabletten

1 Tablette enthält:
500 mg Paracetamol
50 mg HED-3
Sonst. Bestandteile: Mikrokristalline Cellulose, Maistärke, Stearinsäure, Povidon.

### Anwendungsbeispiel 11: Fruchtgummi

| **Inhaltsstoffe** Mengenangaben in Gew.-% | **A** | **B** |
|---|---|---|
| Gelatine | 7.6% | 7.6% |
| Paracetamol | 5% | 5% |
| Symrise-Citrus-Aroma mit Farbstoff | 0.8% | 0.8% |
| HED-1 | 0.5 % | - |
| HED-5 | - | 2.5 % |
| Wasser | Auf 100% | Auf 100% |

Zur Herstellung eines Fruchtgummis mit 200 mg Paracetamol pro Dosis werden die in der obigen Rezeptur gelisteten Inhaltsstoffe zu einer aufgekochten Zuckersirupmischung (89° Brix) zugegeben.

## Patentansprüche

1. Homoeriodictyol (HED) oder Salz davon oder Mischung aus HED und einem oder mehreren Salzen davon oder Mischung aus mehreren Salzen davon zur Anwendung in einem therapeutischen Verfahren zum Reduzieren der Magensäuresekretions-stimulierenden Wirkung von N-Acetyl-4-aminophenol (Paracetamol).

2. HED oder Salz davon oder Mischung aus HED und einem oder mehreren Salzen davon oder Mischung aus mehreren Salzen davon zur Anwendung nach Anspruch 1, zudem zum Maskieren des bitteren Geschmacks von Paracetamol.

3. HED oder Salz davon oder Mischung aus HED und einem oder mehreren Salzen davon oder Mischung aus mehreren Salzen davon zur Anwendung nach Anspruch 1 oder 2 in einer Zubereitung, enthaltend HED und/oder ein oder mehrere Salze davon und Paracetamol, wobei die Menge an HED und/oder Salz(en) davon ausreicht, um die Magensäuresekretions-stimulierende Wirkung von Paracetamol zu reduzieren und, vorzugsweise, zudem ausreicht, um den bitteren Geschmack von Paracetamol zu maskieren.

4. HED oder Salz davon oder Mischung aus HED und einem oder mehreren Salzen davon oder Mischung aus mehreren Salzen davon zur Anwendung nach Anspruch 3, wobei die Konzentration von HED und/oder Salz(en) davon in der Zubereitung im Bereich von 0,000001 bis 30 mM liegt, vorzugsweise im Bereich von 0,00001 bis 3 mM, weiter bevorzugt im Bereich von 0,0001 bis 1 mM, besonders bevorzugt im Bereich von 0,001 bis 0,4 mM.

5. HED oder Salz davon oder Mischung aus HED und einem oder mehreren Salzen davon oder Mischung aus mehreren Salzen davon zur Anwendung nach einem der Ansprüche 3 oder 4, wobei die Konzentration von Paracetamol in der Zubereitung im Bereich von 0,001 bis 1000 mM liegt, vorzugsweise im Bereich von 0,01 bis 800 mM, weiter bevorzugt im Bereich von 0,1 bis 750 mM, besonders bevorzugt im Bereich von 1 bis 600 mM.

6. HED oder Salz davon oder Mischung aus HED und einem oder mehreren Salzen davon oder Mischung aus mehreren Salzen davon zur Anwendung nach einem der vorangehenden Ansprüche, vorzugsweise nach einem der Ansprüche 3 bis 5, wobei das Verhältnis der Gesamtkonzentration an HED und/oder Salz(en) davon zur Gesamtkonzentration an Paracetamol im Bereich von 1 : 100000 bis 1 : 1 liegt, vorzugsweise im Bereich von 1 : 10000 bis 1 : 2, weiter bevorzugt im Bereich von 1 : 5000 bis 1 : 10, besonders bevorzugt im Bereich von 1 : 2000 bis 1 : 20.

7. HED oder Salz davon oder Mischung aus HED und einem oder mehreren Salzen davon oder Mischung aus mehreren Salzen davon zur Anwendung nach einem der Ansprüche 3 bis 5, oder Anspruch 6, sofern rückbezogen auf einen der Ansprüche 3 bis 5, wobei die Zubereitung zudem einen oder mehrere Bestandteile ausgewählt aus der Gruppe bestehend aus Eriodictyol, Phloretin, Hesperetin, 2,4-Dihydrobenzoesäure-*N*-Vanillylamid, 5,7-Dihydroxy-4-(4-hydroxy-phenyl)-chroman-2-on, 5,7-Dihydroxy-4-(4-hydroxy-3-methoxy-phyenyl)-chroman-2-on, 5,7-Dihydroxy-4-(4-pyridyl)chroman-2-on, 7,3-Dihydroxy-4'-methoxyflavan, Lariciresinol und Matairesinol, und deren jeweiligen (wenn anwendbar) einzelnen oder in Gemischen vorliegenden Stereoisomeren (Diasteromere oder Enantiomere) umfasst.

8. HED oder Salz davon oder Mischung aus HED und einem oder mehreren Salzen davon oder Mischung aus mehreren Salzen davon zur Anwendung nach einem der vorangehenden Ansprüche, wobei das bzw. ein, mehrere oder sämtliche Salze von HED ausgewählt ist bzw. sind aus der Gruppe bestehend aus Natrium-, Kalium-, Calcium- und Ammonium-Salzen.

## Claims

1. Homoeriodictyol (HED) or salt thereof or mixture of HED and one or more salts thereof or mixture of several salts thereof for use in a therapeutic method for reducing the gastric acid secretion stimulating effect of N-acetyl-4-aminophenol (Paracetamol).

2. HED or salt thereof or mixture of HED and one or more salts thereof or mixture of several salts thereof for use according to claim 1, additionally for masking the bitter taste of paracetamol.

3. HED or salt thereof or mixture of HED and one or more salts thereof or mixture of several salts thereof for use according to claim 1 or 2 in a preparation comprising HED and/or one or more salts thereof and paracetamol, wherein the amount of HED and/or salt(s) thereof is sufficient to reduce the gastric acid secretion stimulating effect of paracetamol and, preferably, is additionally sufficient to mask the bitter taste of paracetamol.

4. HED or salt thereof or mixture of HED and one or more salts thereof or mixture of several salts thereof for use according to claim 3, wherein the concentration of HED and/or salt(s) thereof in the preparation is in a range of from 0.000001 to 30 mM, preferably in a range of from 0.00001 to 3 mM, further preferably in a range of from 0.0001 to 1 mM, particularly preferably in a range of from 0.001 to 0.4 mM.

5. HED or salt thereof or mixture of HED and one or more salts thereof or mixture of several salts thereof for use according to any of claims 3 or 4, wherein the concentration of paracetamol in the preparation is in a range of from 0.001 to 1000 mM, preferably in a range of from 0.01 to 800 mM, further preferably in a range of from 0.1 to 750 mM, particularly preferably in a range of from 1 to 600 mM.

6. HED or salt thereof or mixture of HED and one or more salts thereof or mixture of several salts thereof for use according to any one of the preceding claims, preferably according to any of claims 3 to 5, wherein the ratio of the total concentration of HED and/or salt(s) thereof and the total concentration of paracetamol is in a range of from 1 : 100000 to 1 : 1, preferably in a range of from 1 : 10000 to 1 : 2, further preferably in a range of from 1 : 5000 to 1 : 10, particularly preferably in a range of from 1 : 2000 to 1 : 20.

7. HED or salt thereof or mixture of HED and one or more salts thereof or mixture of several salts thereof for use according to any of claims 3 to 5, or claim 6 if back-referenced to any of claims 3 to 5, wherein the preparation additionally comprises one or more components selected from the group consisting of eriodictyol, phloretin, hesperetin, 2,4-dihydrobenzoic acid-N-vanillyl amide, 5,7-dihydroxy-4-(4-hydroxyphenyl)-chroman-2-one, 5,7-dihydroxy-4-(4-hydroxy-3-methoxy-phenyl)-chroman-2-one, 5,7-dihydroxy-4-(4-pyridyl)chroman-2-one, 7,3-dihydroxy-4'-methoxy flavane, lariciresinol and matairesinol and their respective (where applicable) stereoisomers (diastereomers or enantiomers) present as single substances or in mixtures.

8. HED or salt thereof or mixture of HED and one or more salts thereof or mixture of several salts thereof for use according to any of the preceding claims, wherein the, one, several or all salts of HED is/are selected from the group consisting of sodium, potassium, calcium and ammonium salts.

## Revendications

1. Homoériodictyol (HED) ou sel de celui-ci ou mélange d'HED et d'un ou de plusieurs sels de celui-ci ou mélange de plusieurs sels de celui-ci destiné à être utilisé dans un procédé thérapeutique pour réduire l'effet stimulant la sécrétion d'acide gastrique de N-acétyl-4-aminophénol (paracétamol).

2. HED ou sel de celui-ci ou mélange d'HED et d'un ou de plusieurs sels de celui-ci ou mélange de plusieurs sels de celui-ci pour l'utilisation selon la revendication 1, en plus pour masquer le goût amer de paracétamol.

3. HED ou sel de celui-ci ou mélange d'HED et d'un ou de plusieurs sels de celui-ci ou mélange de plusieurs sels de celui-ci pour l'utilisation selon la revendication 1 ou 2 dans une préparation contenant de l'HED et/ou un ou plusieurs sels de celui-ci et du paracétamol, dans lequel la quantité d'HED et/ou de sel(s) de celui-ci est suffisante pour réduire l'effet stimulant la sécrétion d'acide gastrique de paracétamol et, de préférence, est suffisante en outre pour masquer le goût amer de paracétamol.

4. HED ou sel de celui-ci ou mélange d'HED et d'un ou de plusieurs sels de celui-ci ou mélange de plusieurs sels de celui-ci pour l'utilisation selon la revendication 3, dans lequel la concentration d'HED et/ou de sel(s) de celui-ci dans la préparation se situe dans la gamme allant de 0,000001 à 30 mM, de préférence dans la gamme allant de 0,00001 à 3 mM, plus préférablement dans la gamme allant de 0,0001 à 1 mM, de manière particulièrement préférée dans la gamme allant de 0,001 à 0,4 mM.

5. HED ou sel de celui-ci ou mélange d'HED et d'un ou de plusieurs sels de celui-ci ou mélange de plusieurs sels de celui-ci pour l'utilisation selon l'une quelconque des revendications 3 ou 4, dans lequel la concentration de paracétamol dans la préparation se situe dans la gamme allant de 0,001 à 1000 mM, de préférence dans la gamme allant de 0,01 à 800 mM, plus préférablement dans la gamme allant de 0,1 à 750 mM, de manière particulièrement préférée dans la gamme allant de 1 à 600 mM.

6. HED ou sel de celui-ci ou mélange d'HED et d'un ou de plusieurs sels de celui-ci ou mélange de plusieurs sels de celui-ci pour l'utilisation selon l'une quelconque des revendications précédentes, de préférence selon l'une quelconque des revendications 3 à 5, dans lequel le rapport de la concentration totale d'HED et/ou de sel(s) de celui-ci à la concentration totale de paracétamol se situe dans la gamme allant 1 : 100000 à 1 : 1, de préférence dans la gamme allant de 1 : 10000 à 1 : 2, plus préférablement dans la gamme allant de 1 : 5000 à 1 : 10, de manière particulièrement préférée dans la gamme allant de 1 : 2000 à 1 : 20.

7. HED ou sel de celui-ci ou mélange d'HED et d'un ou de plusieurs sels de celui-ci ou mélange de plusieurs sels de celui-ci pour l'utilisation selon l'une quelconque des revendications 3 à 5 ou la revendication 6, lorsqu'elle est dépendante de l'une des revendications 3 à 5, dans lequel la préparation comprend en outre un ou plusieurs ingrédients choisis dans le groupe constitué par l'ériodictyol, la phlorétine, l'hespérétine, le N-vanillylamide de l'acide 2,4-dihydrobenzoïque, le 5,7-dihydroxy-4-(4-hydroxyphényl)-chroman-2-one, le 5,7-dihydroxy-4-(4-hydroxy-3-méthoxy-phényl)chroman-2-one, le 5,7-dihydroxy-4-(4-pyridyl)chroman-2-one, le 7,3-dihydroxy-4'-méthoxyflavane, le laricirésinol et le matairésinol, ainsi que leurs stéréoisomères (diastéréomères ou énantiomères) respectifs (si appiquables) présents séparément ou en mélanges.

8. HED ou sel de celui-ci ou mélange d'HED et d'un ou de plusieurs sels de celui-ci ou mélange de plusieurs sels de celui-ci pour l'utilisation selon l'une quelconque des revendications précédentes, dans lequel ledit ou bien un, plusieurs ou tous les sels d'HED est ou bien sont choisi(s) dans le groupe constituté par les sels de sodium, de potassium, de calcium et d'ammonium.
